# EUROPEAN PATENT SPECIFICATION

(11) **EP 2 553 120 B1**
(45) Date of publication and mention of the grant of the patent: **22.04.2015**
(21) Application number: 11719888.7
(22) Date of filing: 29.03.2011
(51) Int. Cl.: C12Q 1/68

(54) **PEPTIDE NUCLEICACID PROBE, KIT AND METHOD FOR DETECTION AND/OR QUANTIFICATION OF SALMONELLA SPP. AND APPLICATIONS THEREOF**
PEPTID-NUKLEINSÄURE-SONDE SOWIE KIT UND VERFAHREN ZUR ERKENNUNG ODER QUANTIFIZIERUNG VON SALMONELLA SPP. UND ANWENDUNGEN DAVON
SONDE D'ACIDE NUCLÉIQUE PEPTIDIQUE, TROUSSE ET PROCÉDÉ POUR LA DÉTECTION ET/OU LA QUANTIFICATION DE SALMONELLA SPP. ET APPLICATIONS CORRESPONDANTES

(30) Priority: 29.03.2010 PT 10005029
(43) Date of publication of application: 06.02.2013
(73) Proprietor: Universidade do Minho, 4700-320 Braga (PT)
(72) Inventor: RIBEIRO PINTO DE OLIVEIRA AZEVEDO, Nuno Filipe, 4710-504 Braga (PT); LOPES DA COSTA VIEIRA, Maria João, 4715-010 Braga (PT); FERNANDES ALMEIDA, Carina Manuela, 4730-305 Vila Verde (PT); KEEVIL, Charles William, Southampton Hampshire SO17 1BJ (GB)
(74) Representative: Ferreira, Maria Silvina
(86) International application number: PCT/IB2011/051337
(87) International publication number: WO 2011/121544

(56) References cited:
- WO-A2-02/068579
- US-B1- 6 664 045
- PERRY-O'KEEFE H ET AL: "Identification of indicator microorganisms using a standardized PNA FISH method.", JOURNAL OF MICROBIOLOGICAL METHODS DEC 2001 LNKD- PUBMED:11714518, vol. 47, no. 3, December 2001 (2001-12), pages 281-292, XP002646480, ISSN: 0167-7012
- ALMEIDA C ET AL: "Development and application of a novel peptide nucleic acid probe for the specific detection of Cronobacter genomospecies (Enterobacter sakazakii) in powdered infant formula.", APPLIED AND ENVIRONMENTAL MICROBIOLOGY MAY 2009 LNKD- PUBMED:19270117, vol. 75, no. 9, May 2009 (2009-05), pages 2925-2930, XP002646481, ISSN: 1098-5336
- DATABASE Genbank [Online] ncbi; 10 October 2002 (2002-10-10), "lle23h11.b1", XP002646482, retrieved from nlm Database accession no. BZ064740

### SEQUENCE LISTING

<110> Universidade do Minho
<120> SONDAS DE ÁCIDO PÉPTIDO NUCLEICO, ESTOJO E MÉTODO PARA DETECTAR SALMONELLA E RESPECTIVAS APLICAÇÕES
<130> PAT 41490/10
<160> 8
<170> PatentIn version 3.5
<210> 1
   <211> 15
   <212> DNA
   <213> Salmonella
<400> 1
   aggagcttcg cttgc 15
<210> 2
   <211> 15
   <212> DNA
   <213> Salmonella
<400> 2
   tcaggagctt cgctt 15
<210> 3
   <211> 15
   <212> DNA
   <213> Salmonella
<400> 3
   ggagcttcgc ttgcg 15
<210> 4
   <211> 17
   <212> DNA
   <213> Salmonella
<400> 4
   tcaggagctt cgcttgc 17
<210> 5
   <211> 15
   <212> DNA
   <213> Salmonella
<400> 5
   aggagctccg cttgc 15
<210> 6
   <211> 14
   <212> DNA
   <213> Salmonella
<400> 6
   taagccggga tggc 14
<210> 7
   <211> 18
   <212> DNA
   <213> Salmonella
<400> 7
   tcgactgact tcagctcc 18
<210> 8
   <211> 18
   <212> DNA
   <213> Salmonella
<400> 8
   aatcacttca cctacgtg 18

## Description

### Field of the invention

This invention relates to a process for the detection of microorganisms relevant for clinical and food safety. For that purpose, a PNA probe for the detection of *Salmonella* genus was developed.

In addition to the probe, the present invention includes the ONA FISH procedure and its application to a kit for the detection and/or quantification of *Salmonella* spp., which can be used in the food and clinical fields.

### Background of the invention

The genus *Salmonella* includes several pathogenic bacteria that can cause diseases that range from simple gastroenteritis to systemic infections. The disease severity is generally determined by the virulence of the *Salmonella* species/strain, the host (human or other animal species)and host health condition.

Phylogenetic analyzes of the genus have shown that it is divided into two species: *Salmonella bongori* and *Salmonella enterica.* Nonetheless, more than 2500 serotypes have been identified to date, the majority of which capable to infect humans and a wide range of animal species. Salmonella strains are conventionally identified and classified according to the Kauffmann-White serotyping scheme, which is based on the type of antigens which are present in the outer membrane.
This bacterium can be transmitted directly from person to person via the fecal-oral route or by direct contact with external reservoirs when fecal contamination of soil, water, and food occurs. It is therefore important to develop a robust detection method enabling the identification of the bacterium in all these sample types. For instance, the "Food and drug Administration" intents to control the transmission of *Salmonella enteritidis* through the implementation of a test program in poultry farms for contaminated eggshells, since eggshells have been identified as important in the transmission of the microorganism.
The detection of *Salmonella* is traditionally performed using culture methods. However, these methods are laborious and rather time-consuming (4 to 6 days) and laborious process. As such, the development of new methods is needed, which are faster and more reliable, that can assist in the control of the bacteria in order to reduce salmonellosis in both people and animals. For this purpose, a number of molecular detection methods have been developed to reduce the time required for the identification of *Salmonella* in food, feces, water and clinical samples. These methods include immunoenzymatic assays (ELISA), polymerase chain reaction (PCR), and FISH. However, some studies showed that PCR- and ELISA-based methods failed to detect some samples that were positive by culture method. Even so, PCR-based methods have proved to be more accurate than the ELISA methods. On the other hand, when a selective enrichment step is performed before PCR, not only all cluture-positive samples are detected, but also the presence of *Salmonella* that was not detected by the culture method can be detected by PCR. These studies reveal that the enrichment step can increase the molecular assay sensitivity by eliminating problems such as the low numbers of bacteria and the presence of inhibitory substances in certain types of samples, such as food and fecal samples. However, PCR-based methods usually require a DNA extraction step, and none of the methods referred to above, except FISH, allows a direct visualization of the bacterium within the sample. This can be important, for instance, by allowing the evaluation of *Salmonella* contamination in eggshells directly in the sample.

FISH is a molecular assay widely applied for bacterial identification and localization within samples. This method is based on the specific binding of small oligonucleotides (probes) to particular rRNA regions. The probe is coupled to a fluorochrome and after the hybridization a fluorescence signal can be detected due to the high rRNA copy number within the cell. There are already some studies reporting *Salmonella* detection using DNA probes (Kutter et *al.,* 2006; Nordentoft et *al.,* 1997).
More recently, peptide nucleic acid probes (PNA) have been developed for microbial detection. These molecules mimic DNA and are capable of hybridizing specifically with complementary nucleic acids obeying to the Watson-Crick rules. However, the established bond is stronger since, in the PNA molecule, its negative charged sugar-phosphate backbone structure is substituted by neutral repeated (2-aminoethil) glyc in units. The adequate use of this molecule in FISH technology has rendered the procedure more robust, quicker and more efficient.
For *Salmonella,* a PNA FISH method has been previously developed (Perry-O'Keefe et *al.,* 2001). However, the probe that was used has a sequence that is complementary to other species, such as *Actinobacillus actinomycetemcomitans, Buchnera aphidicola, Haemophilus influenza* and *Yersinia* spp, making it less attractive for diagnosis. It was thus considered important to design and test a new probe and to develop a new method for this particular microorganism.

It is important to notice that, although very robust after optimized, the development of PNA FISH methods is, just as the development of PCR methods, extremely demanding and requires a great knowledge of the chemical and physical characteristics of the different parameters involved. Furthermore, it is well known tha thaving a PNA FISH method working for an organism does not warrant that other sequences targeting the same organism will function. Moreover, although researchers usually feel a natural lack of interest in publishing negative results, several studies can be found in literature in which the authors were only able to put to work some of the probes for the same microorganism.

### Summary of the Invention

The present invention refers to a peptide nucleic acid (PNA) probe and a method therof for the detection of the *Salmonella* genus (that is, identification or quantification).

The probe described in the present invention recognizes the microorganism 23S rRNA or the genomic sequences corresponding to the mentioned rRNA. The PNA probes have physiochemical characteristics that are inherent to its structure and when they are applied to a FISH-based method, allow a faster, more robust and more specific analysis than using a DNA.
One of the advantages of this method is that the probe works robustly in a wide variety of biological samples, which usually does not happen with the other detection molecular methods.

Another relevant aspect is the time required for detection. The method here developed matches the best times reported for the remaining molecular methods, even when the type of sample requires an enrichment step prior to the analysis. The rapidity and the reliability of the method can determine the appropriate and timely treatment of a contamination and/or infection for both clinical or food security perspectives.

Another aspect of the present invention is related to the development of a kit based on the application of this probe to fluorescence *in situ* hybridization (FISH), allowing the detection of *Salmonellain* a broad range of biological samples, in a prompt and simple way.

In a preferred embodiment of the present invention, the PNA probe here described allow the detection of the target sequence in rRNA, in rDNA or in complementary sequences of the rRNA of *Salmonella.*

In a more preferable embodiment of the present invention, the previously described sequences are linked to at least one type of detectable fraction. The type of detectable fraction to be used may be selected from one of the following groups: a conjugate, a branched detection system, a chromophore, a fluorophore, radioisotope, an enzyme, a hapten or a luminescent compound, among others.

In an even more preferable embodiment, the fluorophore group can be at least one of the following: Alexa series fluorophores, cyanines, 5- (and -6) Carboxy-2', 7'-dichlorofluorescein, 5-ROX (5-carboxy-X-rhodamine, triethylammonium salt), among others.

It is still the subject of the present invention a kit for the detection of the presence or absence and/or quantification of *Salmonella* in biological samples.

In a more preferable embodiment of the present invention, the kit may additionally present at least one of the following solutions: a fixation solution, a hybridization solution and a washing solution.

In yet another preferred embodiment of the present invention, the fixation solution can comprise paraformaldehyde and ethanol, namely 2-8% (weight/vol) of paraformaldehyde and 25-90% (vol/vol) of ethanol and/or hybridization solution may comprise formamide.

It is still the object of the present invention the description of a method for the detection of *Salmonellaor* for the detection of *Salmonellain* biological samples, which uses the PNA probe mentioned earlier and which comprises the following steps:
- contact of the PNA probe with biological samples;
- hybridization of the PNA probe with the target sequence of the microorganisms present within the biological samples;
- detection of the hybridization as an indication of the mentioned detection and quantification in the biological samples, the hybridization may be preferably carried our by fluorescence.

The biological samples can be taken from blood, air, food, water, biopsies or feces, among others.

It is still the object of the present invention the use of the PNA probes described earlier, the use of the kit described earlier and the methodology to be applied in a methodology of detection of *Salmonella,* or detection of *Salmonella* in biological samples.

### General description of the invention

The present invention comprises the PNA probe, reagents, methods and a kit intended for the detection or quantification of *Salmonella* strains.

The PNA probe herewith described allows the specific detection of the *Salmonella* genus through the bonding to rRNA, genomic sequences corresponding to rRNA (r), or their complementary sequences.

The higher specificity of the PNA probes (in relation to DNA probes) allows a better discrimination between related nucleotide sequences.

This has particular relevance to this probe since there are some phylogenetically related microorganisms to *Salmonella* that present only one mismatch (nucleotide at position 14 of the probe described in this invention) within the selected target region. Some examples of this situation are the species of *Shigella,* the species *Yersinia enterocolitica* and the strain *Escherichia coli* K12.

The PNA probe described in this invention has 15 nucleotides with the following nucleotidic sequence:
SEQ ID No. 1 - 5'-AGG AGC TTC GCT TGC-3'.

This probe is applied to the analysis by fluorescence *in situ* hybridization (FISH), which, in the case of *Salmonella-*positive samples, results in the emission of detectable fluorescent signal either through fluorescence microscopy or through flow cytometry.

The development of the new PNA-FISH probe was carried out empirically using specific software. The selection of the probe sequence was initially performed by aligning rDNA sequences of the target microorganism, with sequences of related microorganisms. This allowed the identification of potentially useful regions, which will be then evaluated based on other parameters such as specificity, hybridization temperature, percentage of guanine/cytosine, bond free energy and secondary structure.

After the probe design and synthesis, the three steps of FISH procedure, fixation/permeabilization, hybridization and wash, have to be developed and optimized for the selected probe. This process usually involves the following parameters: temperature, concentration of formamide and ethanol, and hybridization and washing times. It is important to notice that due to the process complexity and the large number of variables, is not always possible to develop a method for each sequence and as such, several alternatives and sequences are often tested.

A well-succeeded hybridization afterwards allows inferring about the presence/absence and even the concentration of a microorganism by fluorescence microscopy, flow cytometry or real time PCR.T he detected fluorescent signal is generally the result of the specific binding of the small probes to tens or hundreds of rRNA copies present in the bacteria cytoplasm. That detectable fraction of the probe, which reports the existence of a stable complex formed by the probe and the target, is selected from one of the following groups: a conjugate, a branched detection system, a chromophore, a fluorophore, radioisotope, an enzyme, a hapten or a luminescent compound.

The method described in the present invention comprises contact of a sample with at least one PNA probe with a similar sequence to that previously described. Consequently, the analysis is based on a single test with a definitive result in opposition to the conventional methods for the detection of *Salmonella* that are based on phenotypic feature sand require several days to yield the result.

It is still the object of the present invention a kit suitable for carrying out the test to detect, i.e., to find, identify or measure the *Salmonella* presenting biological samples. The kit comprises the PNA probe and other selected reagents or compounds needed to perform *in situ* hybridization tests.

In a more preferable realization, the kit suitable for performing the assay for the detection, identification or quantification of *Salmonella* comprises additionally a fixation, hybridization and wash solution.

Preferably, the method intends to be a diagnostic adjuvant for therapeutic decision and quality control. Thus, the implementation of this method for *Salmonella* identification will allow the adequate clinical treatment to the bacterium and the early identification of the contamination source.

The PNA probes can be applied directly on the sample prepared on a slide, since the application of these probes doesn't involve the use of reagents or enzymes for the permeabilization of the cellular membranes before the hybridization. However, some of the compounds that are frequently used in hybridization are required.
Therefore, the probes are normally included in more user-friendly kits.
If the desired approach involves the PNA FISH analysis by flow cytometry, the probe could be applied to the sample in suspension, using the same hybridization compounds.

### Detailed description of the invention

### I- Definitions

a) As used herein, the term "nucleotide" includes natural and artificial molecules known generally by those who use technology related with nucleic acids, to thereby generate polymers that bind specifically to nucleic acids;
b) When used the term "nucleotide sequence" is the same as referring to a segment of a polymer containing subunits, in this case the nucleotides;
c) The term "target sequence" refers to a nucleotide sequence of *Salmonella* that is intended to be detected in the test, where the portion of nucleotides of the probe is designed to hybridize;
d) The term "PNA probe" refers to a polymer of subunits of PNA which has a nucleotide sequence and is specific to hybridize with a target sequence of the microorganism of interest. PNA molecules are DNA mimics in which the negatively charged sugar-phosphate backbone structure is replaced by an a chiral and electrically neutral formed by repeated N - (2-aminoethyl) glycine units;
e) When using the term "detectable fraction", it refers to molecules that can be connected to the probe, to thereby render the probe detectable by an instrument or method;
f) The term "sample" refers to any biological sample that may contain the microorganism or target sequence for detection. The samples could be clinical (e.g. blood, urine, feces, etc.), food (e.g. meat, eggs, infant formulae, milk, etc.) or environmental (e.g. water).

### II - Brief Description of the Drawings

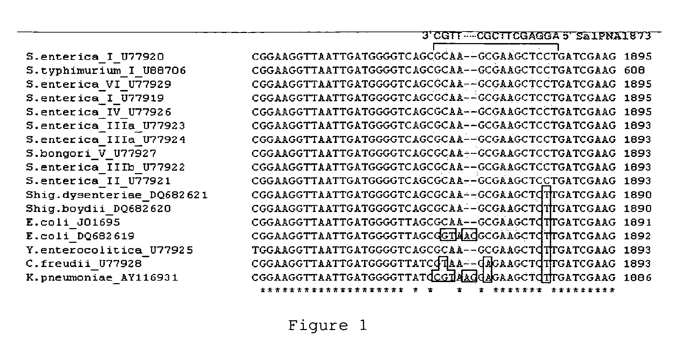

Figure 1 presents the partial alignment of the 23S rRNA sequences for probe selection. The complementary sequence of the SalPNA1873 probe is shown above the alignment and the polymorphic positions are marked as well.

### III - Description

### PNA probe design:

To identify potentially useful oligonucleotides to use as probe, seventeen 23S rRNA gene sequences available at the National Centre for Biotechnology Information (NCBI) website (http://www.ncbi.nlm.nih.gov/BLAST/) were chosen. This selection contained ten *Salmonella* sequences, including representative strains of each of the seven subspecies, and seven other strains from related species belonging to *Enterobacteriaceae* family (Figure 1). The sequences were aligned using the ClustalW software available at the European Bioinformatics Institute (EBI) and regions of interest were selected. Six potentially useful regions were identified at that were afterwards tested in NCBI(BLAST software) and in the SILVA rRNA database project (software Probe Check), in order to find the probe with the highest number of *Salmonella* sequences detected and the lowest number of *non-Salmonella* sequences detected. Other criteria were also used for the selection of the probe such as: high Guanine/Cytosine percentage; the type of secondary structures and hybridization temperature.
Only one region of 18 bp appeared capable of detecting all the *Salmonella* strains in the NCBI database. For this region, four possible probes were designed. One of these was selected since it did not hybridize with any *non-Salmonella* sequence and it contained 60% of GC bases. According to the mentioned selection criteria, the chosen sequence was: 5'-AGGAGCTTCGCTTGC-3*'*. This sequence hybridizes between positions 1873 and 1887 of the 23S rRNA of strain *S. enterica* subsp. enterica serovar Typhimurium LT2 (ATCC 43971), accession number U77920. The probe was named SalPNA1873 due to the initial position of the target sequence in strain LT2. Subsequently, the chosen sequence was synthesized and the oligonucleotides were attached, at the N terminus, to the fluorochrome Alexa Fluor594.

### Theoretical evaluation of the PNA probe performance:

After the design of the probe, its performance was evaluated by determining the theoretical values for sensitivity and specificity.These parameters were evaluated with the above mentioned software ProbeCheck available in the rRNA SILVA databases. For this theoretical estimation all 101 *Salmonella* sequences in the databases were considered (including only the good-quality sequences with at least 1,900 bp). The probe was aligned with a total of 11,124 sequences present in the database for the large RNA subunit (LSU,23S/28S). It was also tested against the database for the small subunit (SSU,16S/18S) to assess the existence of possible cross-hybridization with the 16S rRNA sequences. Specificity was calculated as nSs/(TnS) x 100, where nSs stands for the number of *non-Salmonella* strains that did not react with the probe and TnS is the total number of *non-Salmonella* strains examined. Sensitivity was calculated as Ss/(TSs) x 100, where Ss stands for the number of *Salmonella* strains detected by the probe and TSs is the total number of *Salmonella* strains present in the database.
The search confirmed that the probe SalPNA1873 only detected the 101 *Salmonella* spp. sequences existing in the database. Therefore, a theoretical specificity and sensitivity of 100% were obtained (Table 1).
In order to compare the probe developed in this study with probes developed earlier, the theoretical specificity and sensitivity of the probes were tested:
- Sal23S10 (Perry-O'Keefe et al., 2001)- SEQ IDNo. 6;
- Salm63 (Kutter et al., 2006) - SEQ ID No. 7;
- Sal3 (Nordentoft et al., 1997) - SEQ ID No. 8
were also assessed with the ProbeCheck software (Table 1).

The theoretical assessment of these sequences showed that the probes Seq. ID N° 6, Seq. ID N° 7 and Seq. ID N°8, detected 95, 73 and 96 sequences of Salmonella, respectively, of the 101 strains existing in the database, which correspond to sensitivity values of 94%, 72% and 95%. Regarding the number of *non-Salmonella* sequences detected, the Seq ID No. 1 and Seq. ID No. 8 did not detect any sequence, unlike Seq. ID No. 6 and Seq. ID No. 7. These values allowed estimating specificities of 100% (Seq ID No. 1 and Seq. ID No. 8), 98.13% (Seq ID No. 6) and 99.97% (Seq ID No.7).
The theoretical evaluation performed showed that the probe SalPNA1873 improves the detection of *Salmonella* mainly due to two aspects: the advantages of the PNA molecule and the specificity and sensitivity values of the probe described in this document. The PNA molecule makes the FISH procedure easier and faster when compared to the DNA FISH procedure for probes sal3 and Salm63, while none of the existing PNA probes exceeds the theoretical values of specificity and sensitivity obtained for the probe SalPNA1873.

**Table 1 - Theoretical specificities and sensitivities of the existing probes for detection of Salmonella spp.**

| Probe | Sal3 SEQ No.8 | Salm63 SEQ No.7 | Sal23S10 SEQ No.6 | SalPNA1873 SEQ No. 1 |
|---|---|---|---|---|
| Type | DNA | DNA | PNA | PNA |
| Sequence(5'· 3') | AATCACTTCACC TACGTG | TCGACTGACTTCA GCTCC | TAAGCCGGGAT GGC | AGGAGCTTCGC TTGC |
| N° of *Salmonella* strains detected | 96* | 73* | 95* | 101* |
| N° of non-*Salmonella* strains detected | 0# | 3# | 206# | 0# |
| Specificity(%) | 100 | 99,97 | 98,13 | 100 |
| Sensitivity(%) | 95,05 | 72,23 | 94,06 | 100 |
| Reference | Nordentoft et *al.,* 1997 | Kutter et *al.,* 2006 | O' Keefe et *al.,* 2001 | This work |

| | | | | |
|---|---|---|---|---|
| ** Salmonella* strains detected in a total of 101 *Salmonella* sequences present in the database. # *Non-Salmonella* strains detected in a total of 11,023 *non-Salmonella* sequences deposited in the database. | | | | |

The PNA probe of this invention comprises preferably 15 nucleotides and is identical to the sequence SEQ ID No. 1 - 5'- AGG AGC TTC GCT TGC-3'.

Alternatively, this invention also contemplates variations of the nucleotide sequences of the probes.

### Detectable fraction of the PNA probe:

Not limited to the following examples, the detectable fraction of PNA probe can include various types of molecules such as dextran conjugates, chromophores, fluorophores, radioisotopes, enzymes, haptens, chemiluminescent compound, among others.
As an example, among the fluorophores class those that are preferable for use are (but not limited to): Alexa series fluorophores, Alexa Fluor series, cyanines, 5 - (and -6) Carboxy-2',7'-dichlorofluorescein, 5-ROX (5-carboxy-X-rhodamine, triethylammonium salt.

### Method:

The present invention presents a method for determining the presence of *Salmonella* using a nucleotide sequence with at least 86% of homology with the region of 15 nucleotides here described - SEQ ID No. 1.
The method can include the contact of a sample with PNA probe described herein with the bacterium target sequence inappropriate hybridization conditions or appropriate *in situ* hybridization conditions (as shown in EXAMPLE 1).
The method can be divided into: sample preparation (which includes the enrichment step, when necessary), fixation, hybridization, wash and visualization of the results (see EXAMPLE 1).
The method can be performed on adhered or suspended cells.

### Hybridization conditions - Optimization:

The following steps area possible optimization of the hybridization conditions, without being a limitation of this invention:
There are several factors that influence the hybridization of the PNA probe and the target sequence. These include the percentage of formamide (or other denaturing chemical reagent), salt concentration and consequently the ionic strength, temperature of hybridization, the detergent concentration, pH and others.

To determine the optimal hybridization conditions it may be necessary to fix the different factors and change each factor individually until a desirable discriminatory degree is achieved.
The closer a target sequence is from another non-target in the sample, the greater the stringency degree needed to define the various factors that influence the hybridization. In this invention non-target sequences, such as the *Shigella* species, can have only one different nucleotide in comparison to the target sequences, and as such an increased level of discrimination is necessary to avoid non-specific hybridizations.

For the probes described in this document, the following conditions were detected:
- Hybridization temperatures between 53 °C and 59 °C. The strongest fluorescent signal was obtained at 57 °C, for both hybridization in slides and hybridization in suspension.
- Fixation step using ethanol concentrations ranging between 50% and 80%, but no differences were observed in the signal intensity.
- The hybridization time was tested (30, 45, 60 e 90 min) but the shorter times were as efficient as the longer.

After the optimization of all the parameters referred above, the procedure that was found to result in a stronger fluorescent signal was as follows:
Smears of each bacterial culture were prepared in appropriated slides for fluorescence microscopy observation. Then the smear were:
   - Immersed in 4% (wt/vol) paraformaldehyde (Sigma)for 10 minutes, followed by 50% (vol/vol) ethanol, also for 10 minutes;
   - The samples were air-dried and then covered with 20 µl of hybridization solution containing: 10% (wt/vol) dextran sulfate (Sigma);10 mM NaCl (Sigma);30% (vol/vol)formamide (Sigma);0.1% (wt/vol) sodium pyrophosphate (Sigma);0.2%(wt/vol) polyvinylpirrolydone (Sigma);0.2% (wt/vol) Ficoll (Sigma);5 mM disodium EDTA (Sigma);0.1% (vol/vol) Triton X-100 (Sigma);50 mM Tris-HCl (pH 7.5; Sigma) and 200nM of PNA probe;
   - The samples were covered with cover slips placed in small wet boxes protected from light and incubated for 30 minutes at 57 °C;
   - Afterwards, the cover slips were removed and the slides were submerged in a pre-warmed wash solution (57°C) containing 5 mM Tris Base (Sigma), 15 mM NaCl (Sigma) and 1 % (vol/vol)Triton X-100 (pH 10; Sigma).
   - The washing step was also carried out for 30 minutes at 57°C. Subsequently, the slides were removed from the wash solution and dried at 57 °C in the same incubator for approximately 5 minutes.
   - Before the microscope observation, a drop of non-fluorescent immersion oil (Merck) was placed and covered with a cover slip. The slides were stored in the dark for a maximum of 24 hours before microscopy.

The hybridization can also be performed in suspension. In some cases this procedure helps almost totally eliminating the auto fluorescence, namely the auto fluorescence of the erythrocytes, in the case of blood samples, and the auto fluorescence of proteins in infant formulas. In this case the culture homogenized in sterile water is centrifuged (10,000xg for 5 minutes) and the pellet is homogenized in 500µl 4% paraformaldehyde. After 1 hour, the cells centrifuged once again, in order to remove the paraformaldehyde, and the pellet is homogenized in 500 µl of 50% (vol/vol) ethanol. After 30 min of incubation at -20°C, the cells are homogenized once again in 100 µl of hybridization solution with 200 nM PNA probe and incubated at 57°C for 30 min. After hybridization, the cells were centrifuged and homogenized in 500 µl of wash solution (as described above) and incubated at 57°C for 30 min. Finally, the cells are centrifuged to remove the wash solution and homogenized in 500 µl of sterile water. Next, 20µl of the cell suspension are spread on microscope slides suitable for fluorescence or 200 µl are filtered through a membrane (pore size 0.2 µm, cellulose nitrate, Whatman).
In order to check that the signal that was obtained was not related with auto fluorescence, all samples were observed with the other filters available in the microscope. The samples were also simultaneously stained with DAPI to confirm that all cells present in the sample were marked with the probe SalPNA1873 - SEQ ID No. 1 (described in the present invention). Further, a negative control was performed in each assay, following all the steps of the procedure but without the addition of probe to the hybridization solution.

### Testing of the probe experimental specificity and sensitivity.

Once the hybridization method was fully optimized, the experimental values of specificity and sensitivity of the PNA probe were tested.
For this, the procedure was applied to 61 representative *Salmonella* strains (belonging to the two *Salmonella* species and to the six *S. enterica* subspecies) and to 46 other strains. The latter strains included 25 taxonomically related strains belonging to the same family *(Shigella, Klebsiella, Citrobacter, Pantoea, Yersinia, Enterobacter, Escherichia* and *Serratia)* and 21 strains belonging to different orders *(Pseudomonas),* classes *(Helicobacter* and *Campylobacter*), oreven phylum *(Listeria* and *Staphylococcus).* Apart from S. *enterica* subsp. VI that was not detected by SalPNA1873 - SEQ ID no. 1, all remaining 59 *Salmonella* strains were detected, whereas no hybridization was observed for the other species used. It was not possible to assess the PNA-FISH result for three *Shigella* species, due to the strong auto fluorescence signal of these strains detected in both positive and negative (without probe) samples. This result is not related to the difference of only one nucleotide between the probe and the *Shigella* species because *Shigella flexneri,* which has a similar RNA sequence, did not hybridize with the probe. Moreover, other species such as *Y. enterocolitica* and *Escherichia coli* K-12 also have only one mismatch in exactly in the same position, and no cross-reaction was observed. This result supports the observation from other authors who state that PNA probes allow to distinguish sequences with only one nucleotide mismatch (Petersen et *al.,* 2004). On the basis of these results, an experimental specificity of 100% and sensitivity of 96.7% were obtained.

### Enrichment:

The samples to be analyzed can be obtained from food, biopsies, blood, water, feces among others.
The samples containing *Salmonella* usually present low contamination levels. Because of this, an enrichment step that facilitates the detection process is recommended. This enrichment step can be performed using several types of culture media, from complex rich media to selective media for *Enterobacteriacea* e and/or *Salmonella.* The incubation temperature may depend on the chosen culture medium and the recommended incubation time should be from 8 to 24 hours.

The PNA probe described in this invention was tested in four different types of samples: water, feces, infant formula and blood. The samples were enriched according to the procedure commonly used in the analysis of each type of sample. In the case of water and fecal samples, the first pre-enrichment step was performed as recommended by the International Organization for Standardization (ISO), using buffered peptonated water (BPW) (16 to 18 hours).After this enrichment, the probe allowed the detection of *Salmonella* in all tested samples and the results were consistent with those obtained by the ISO methods recommended for these samples, ISO 6579:2002(Detection of *Salmonella* in food and animal feed) and ISO 6340:1995 (Water quality - Detection and enumeration *Salmonella),* for fecal and water samples, respectively.
In the case of infant formula, the latter was dissolved in water (1:10) and incubated for 8 hours at 37°C as previously suggested for the detection of *Enterobacter sakazakii* in the same type of samples. In the case of blood samples, an enrichment was performed of 16-18 hours at 37 °C in a medium commonly used in blood cultures (TSB - Trypit case Soy Broth). After the enrichment, the bacteria detection was performed in slides or suspension. Before hybridization, the samples should be diluted 1 to 10 in distilled sterile water, in order to minimize the interference of auto fluorescence of some components (as for example the proteins of the infant formula or the erythrocytes).All samples containing *Salmonella* were positive using the PNA probe described in this invention.
These experiments have shown that the detection method using this PNA probe was generally able to detect *Salmonella* in different types of samples in less than 20 hours. Moreover, it was shown that samples containing only 1 CFU per ml can be detected performing an enrichment step of only 8 hours. Although the recommended enrichment media for each type of sample had been tested, it is possible to standardize the enrichment step. Several authors have been showing that the buffered peptonated water (BPW) can be used as a universal culture medium for the enrichment of samples containing *Salmonella,* even for samples with high levels of competing microorganisms.
The comparison of this method to the conventional methods used or the samples mentioned above, showed that the implementation of the PNA sample can save at least 3 days in the detection of bacteria of the *Salmonella* genus.
In the case of solid and compact samples, these should be subjected to a mechanical process of impact with flat blades, that disintegrates the sample, releasing the bacteria to the buffered peptonated water. In the case of biopsies, the samples are cut into 3 to 5 ·m slices, placed on slides and hybridized directly without the need of enrichment steep. Most methods developed for the detection of *Salmonella* are based on PCR techniques or on selective culture media. While the former are more technically demanding and usually also involve an enrichment step to improve the detection limit, the latter are time-consuming and can give inaccurate results (12, 34, 38). The PNA-FISH protocol presented in this work is technically less demanding than the PCR methods and faster and more precise than the culture methods. Although the enrichment step is needed, the total time required for obtaining the result is less than 20 h (except for PIF, which takes only 12 h), a value similar to or even better than the those reported for PCR-based methods (9, 12, 35, 39).
The method here described demonstrated to be a reliable alternative to the currently used culture-based techniques, to the existing *Salmonella* probes, and even to PCR and ELISA protocols. It was shown that the PNA probe here described: allows saving 3 days in the detection when compared with the conventional culture techniques, presents a higher specificity than the previously described probes whether they are DNA or PNA (see Table 1),and is not affected by the presence of substances that act as inhibitors in some molecular approaches, namely PCR.

### Visualization of the results:

This step can be performed in any epifluorescence microscope with a filter sensitive to fluorophore used. Other filters present in the microscope, which are not able to detect the fluorescent signal of the probe, were used to confirm the absence of auto fluorescence.

### Kit:

The present invention also refers to a kit that allows testing for the presence of bacteria from the *Salmonella* genus.
The kit of the present invention comprises a PNA probe identical to SEQ ID No. 1 and another reagents or compositions that are selected to perform the test.
The PNA probes, its characteristics, the methods and kit of this invention are suitable for the analysis of nucleic acids sequences present, or not, internally in the target organism. As such, this invention can be used for both, analysis of the organism or analysis of nucleic acids extracted or derived from the organism of interest, implying that the source of the target sequence is not a limitation on this invention.

The following examples illustrate different situations and several steps for implementing the invention, are preferred embodiments of the present invention, without intending to limit any of them:

### EXAMPLE 1: Detection of bacteria belonging to Salmonella genus indifferent samples (clinical, food or environmental samples)

### Sequence:

SEQ ID No. 1 - 5'-AGG AGC TTC GCT TGC-3 (coupled to Alexa Fluor 594)

### Sample preparation:

The clinical, food or environmental samples were previously subjected to an enrichment step before application of the PNA probe. This happens because usually *Salmonella* presents low contamination levels. This enrichment step can be performed using the medium recommended for the conventional analysis method used for each sample. In case of food, animal feed, feces or water, BPW was used. For powdered milk or infant formulas, sterile water was used to dissolve the powder in a1/10 (weight/vol) ratio. For blood samples, the complex TSB medium was used, commonly used in blood cultures.
Incubation times ranged between 8 (milk powder and infant formula) and 16-18 hours (feces, water, blood, etc.) at 37 °C, at 120 rpm. For the simultaneous analysis of different sample types, it is recommended that the used method is standardized by performing an enrichment in BPW for 8 hours at 37 °C, 120 rpm. In case of negative results after 8 hours of incubation, the PNA FISH analysis should be repeated after an overnight enrichment (16-18 hours). Following the enrichment step, a drop of the culture medium was placed in a slide suitable for fluorescence. The slide was placed for approximately 5 minutes in an incubator oven at 57°C or left to air dry. Samples that do not require an enrichment step should start the procedure in the fixation step.

### Fixation:

For preventing the loss of 23S rRNA during the hybridization process, the samples were immersed in a solution of 4% paraformaldehyde (wt/vol) and 50% ethanol(vol/vol)for 10 minutes each.

### Hybridization:

After fixation, samples were then covered with a drop of hybridization solution containing: 10% (wt/vol) dextran sulfate (Sigma); 10 mM NaCl (Sigma); 30% (vol/vol) formamide (Sigma); 0.1% (wt/vol) sodium pyrophosphate (Sigma); 0.2%(wt/vol) polyvinylpirrolidone (Sigma); 0.2% (wt/vol) Ficol (Sigma); 5 mMdi sodium EDTA (Sigma); 0.1% (vol/vol) Triton X-100 (Sigma); 50 mM Tris-HCl (pH 7.5; Sigma);and 200nM PNA probe. The samples were covered with cover slips (to assure an homogeneous spreading of the probe) placed in small wet boxes (to prevent the evaporation of the hybridization solution) protected from light and incubated for 30 minutes at 57 °C.

### Wash:

After the hybridization time, the cover slips were removed and the slides were immersed in a wash solution pre-warmed at 57°C containing 5 mM Tris Base, 15 mM NaCl and 1 % (vol/vol)Triton X-100 (pH 10). The samples were then placed in an oven at the hybridization temperature for 30 minutes. Subsequently, the slides were removed from the wash solution and dried at 57 °C, in the same incubator, for approximately 5 minutes. Before the microscope visualization, a drop of non-fluorescent immersion oil (Merck) was placed and covered with a cover slip. The slides were kept in the dark for a maximum period of 24 hours before microscopy.

### Results:

The results were obtained through the observation in a fluorescence microscope with a filter capable of detecting the fluorochrome Alexa Fluor 594 bonded to the PNA probe.

### EXAMPLE 2: Detection of bacteria of the generaSalmonella and Cronobacter in infant formulas.

This example illustrates the possibility of using the probe for *Salmonella* spp. together with the probe for *Cronobacter* spp. previously developed in Almeida et *al. ,* 2009. These two genera were recently identified as the most frequent contaminants of powdered infant formulas, which contamination is a major cause of bacteremia, meningitis and necrotizing enterocolitis in newborn infants. These two probes present very similar hybridization temperatures and can be used easily in a multiplex assay (simultaneous usage of several probes).

### Sequences:

SEG ID No. 1 - 5'-AGG AGC TTC GCT TGC-3 (coupled to Alexa Fluor 594)
SEG ID No. 6 - 5'-TGC AGG ATT CTC TGG-3'(coupled to Alexa Fluor 488)

### Preparation of samples:

10 g of each infant formula were weighed and hydrated with 90 ml of sterile water. Higher amounts of infant formula can be used, but keeping the ratio 1/10 (weight/vol). Subsequently, the hydrated samples were incubated for 8 hours at 37°C and 120 rpm. After enrichment, samples were taken and diluted 1/10 to minimize the interference of auto fluorescence from the infant formula proteins. Finally
One drop of the diluted sample was placed in a suitable slide and air dried in a incubator at 57 °C for approximately 5 minutes

### Hybridization:

The hybridization was performed as described previously in Example 1 with one slight difference. The hybridization solution contained two probes: a PNA probe to detect *Salmonella* and a PNA probe to detect *Cronobacter,* each in a concentration of 200nM.

### Wash:

The wash was performed according to the procedure described in Example 1.

### Results:

The results were obtained by observing the samples with a fluorescence microscope equipped with appropriated filters to detect the fluorochromes Alexa Fluor 594 and 488 connected to the PNA probes.

### References:

- Almeida, C., N. F. Azevedo, C. Iversen, S. Fanning, C. W. Keevil, and M. J. Vieira, 2009. Development and application of a novel peptide nucleic acid probe for the specific detection of Cronobacter genomospecies (Enterobacter sakazakii) in powdered infant formula. Appl Environ Microbiol 75:2925-30.
- Perry-O'Keefe, H., S. Rigby, K. Oliveira, D. Sorensen, H. Slender, J. Coull, and J. J. Hyldig-Nielsen, 2001. Identification of indicator microorganisms using a standardized PNA FISH method. Journal of Microbiological Methods 47:281-292. - Kutter, S., A. Hartmann, and M. Schmid. 2006. Colonization of barley (Hordeum vulgare) with Salmonella enterica and Listeria spp. Fems Microbiology Ecology 56:262-271.
- Nordentoft, S., H. Christensen, and H. C. Wegener. 1997. Evaluation of a fluorescence-labelled oligonucleotide tide probe targeting 23S rRNA for in situ detection of Salmonella serovars in paraffin-embedded tissue sections and their rapid identification in bacterial smears. Journal of Clinical Microbiology 35:2642-2648.
- WO 02/27036 A2 - Probes, probe sets, methods and kits pertaining for the detection, identification and/or enumeration of bacteria.

## Claims

1. PNA probe for the detection and/or quantification of *Salmonella* **characterized in that** it comprises SEQ ID No. 1 - 5'-AGG AGC TTC GCT TGC-3'.

2. PNA probe according to claim 1, for detecting the target sequence in rRNA, rDNA or the sequences complementary to *Salmonella* rRNA.

3. PNA probe according to any of the claims 1 to 2, **characterized in that** it is connected to at least one type of detectable fraction, selected from one of the following groups: a conjugate, a branched detection system, a chromophore, a fluorophore, radioisotope, an enzyme, a hapten or a luminescent compound.

4. PNA probe according to claim 3, wherein said fluorophore group is at least one of the following: fluorophores of the Alexa series, Alexa Fluor series, cyanines, 5-(and-6) Carboxy-2', 7'-dichlorofluorescein, the 5-ROX (5-carboxy-X-rhodamine, triethylammonium salt).

5. Kit for detection of *Salmonella,* comprising at least one of the PNA probes described in any one of the claims 1 to 4.

6. Kit according to claim 5, further comprising at least one of the following solutions: one fixation solution, one hybridization solution and one washing solution.

7. Kit according to claim 6, wherein said fixation solution comprises paraformaldehyde and ethanol, preferably 2-8% (wt/vol) of paraformaldehyde and 25-90% (vol/vol) of ethanol.

8. Kit according to claim 6, wherein said hybridization solution comprises formamide.

9. A method for detection of *Salmonella,* **characterized in that** it uses the PNA probes described in any one of the claims 1 to 4 and it comprises the following steps:
- PNA probe contact with the biological samples;
- PNA probe hybridization with the target sequence of the microorganisms present in the referred samples;
- Hybridization detection as indication of the referred detection and quantification of the referred samples;
wherein PNA probe hybridization is performed by covering the biological sample with hybridization solution comprising: 10% (wt/vol) dextran sulfate; 10 mM NaCl; 30% (vol/vol) formamide; 0.1% (wt/vol) sodium pyrophosphate; 0.2% (wt/vol) polyvinylpirrolydone; 0.2% (wt/vol) Ficoll; 5 mM disodium EDTA; 0.1% (vol/vol) Triton X-100; 50 mM Tris-HCl, pH 7.5; and 200nM of PNA probe according to any one of the claims 1 to 4.

10. Method according to claim 9, wherein the biological sample is derived from blood, air, food, water or biopsies and does not contain other than Salmonella nucleic acids comprising the target sequence for the PNA probes described in any one of the claims 1-4.

11. Method according to any one of the claims 9 to 10, wherein in the biological sample is immersed in a fixation solution comprising paraformaldehyde and ethanol, preferably 2-8% (wt/vol) of paraformaldehyde and 25-90% (vol/vol) of ethanol.

12. Method according to claim 9, wherein PNA probe hybridization is performed at a temperature between 53°C and 59°C, preferably 57°C.

13. Method according to any one of the claims 9 to 12, wherein after the PNA probe hybridization there is a washing step performed with a pre-warmed solution comprising 5 mM Tris Base; 15 mM NaCl; and 1 % (vol/vol) Triton X-100, pH 10.

14. Method according to claim 9, wherein the hybridization detection occurs by fluorescence.

15. PNA probes as described in any one of claims 1 to 4, for use in the detection of *Salmonella* spp. in biological samples.

16. The kit as described in any one of claims 5 to 8, for use in the detection of *Salmonella* spp. in biological samples.

## Patentansprüche

1. PNA-Sonde für die Detektion und/oder die Quantifizierung von *Salmonella,* **dadurch gekennzeichnet, dass** sie SEQ ID Nr.: 1-5'-AGG AGC TTC GCT TGC-3` umfasst.

2. PNA-Sonde nach Anspruch 1, zur Detektion der Zielsequenz in rRNA, rDNA oder komplementären Sequenzen zu *Salmonella-rRNA.*

3. PNA-Sonde nach einem der Ansprüche 1 bis 2, **dadurch gekennzeichnet, dass** sie wenigstens mit einer Art von detektierbarem Anteil verbunden ist, der aus einer der folgenden Gruppen ausgewählt wird: einem Konjugat, einem verzweigten Detektionssystem, einem Chromophor, einem Fluorophor, einem Radioisotop, einem Enzym, einem Hapten oder einer lumineszierenden Verbindung.

4. PNA-Sonde nach Anspruch 3, worin die Fluorophor Gruppe wenigstens eine der folgenden ist: Fluorophore der Alexa-Serie, Alexa-Fluor-Serie, Cyanine, 5-(und-6)Carboxy-2',7'-Dichlorfluoresceinlösung, das 5-ROX (5-Carboxy-X-Rhodamin, Triethylammoniumsalz).

5. Kit zur Detektion von *Salmonella,* **dadurch gekennzeichnet, dass** es mindestens eine der in einem der Ansprüche 1 bis 4 beschriebenen PNA-Sonden, umfasst.

6. Kit nach Anspruch 5, **dadurch gekennzeichnet, dass** es ferner mindestens eine der folgenden Lösungen umfasst:
eine Fixierungslösung, eine Hybridisierungslösung und
eine Waschlösung.

7. Kit nach Anspruch 6, worin die Fixierungslösung Paraformaldehyd und Ethanol, bevorzugt 2% - 8 % (Gew./Vol.) Paraformaldehyd und 25% - 90% (Vol./Vol.) Ethanol, enthält.

8. Kit nach Anspruch 6, worin die Hybridisierungslösung Formamid umfasst.

9. Ein Verfahren zum Detektion von *Salmonella,* **dadurch gekennzeichnet, dass** es die in einem der Ansprüche 1 bis 4 beschriebenen PNA-Sonden anwendet und es die folgenden Schritte umfasst:
- PNA-Sonden Kontakt mit den biologischen Proben;
- PNA-Sonden Hybridisierung mit der Zielsequenz, der in den genannten Proben vorhandenen Mikroorganismen;
- Hybridisierungsdetektion als Indiz für die genannten Detektion und Quantifizierung der genannten Proben;
worin die Hybridisierung der PNA-Sonde durch die Abdeckung der biologischen Probe mit einer Hybridisierungslösung enthaltend: 10% (Gew./Vol.) Dextransulfat; 10 mM NaCl; 30% (Vol./Vol.) Formamid; 0,1% (Gew./Vol.) Natriumpyrophosphat; 0,2% (Gew./Vol.) Polyvinylpyrrolidon; 0,2% (Gew./Vol.) Ficoll; 5 mM Dinatrium-EDTA; 0,1% (Vol./Vol.) Triton X-100; 50 mM Tris-HCl, pH 7,5, und; 200 nM an PNA-Sonde nach einem der Ansprüche 1 bis 4, durchgeführt wird.

10. Verfahren nach Anspruch 9, worin die biologische Probe aus Blut, Luft, Nahrung, Wasser oder Biopsien abgeleitet wird und keine anderen Nukleinsäuren enthaltend die Zielsequenz für die in einem der Ansprüche 1 - 4 beschriebenen PNA-Sonden, enthält.

11. Verfahren nach einem der Ansprüche 9 bis 10, worin die biologische Probe in eine Fixierungslösung die Paraformaldehyd und Ethanol, bevorzugt 2% - 8% (Gew./Vol.) Paraformaldehyd und 25% - 90% (Vol./Vol.) Ethanol enthält, eingetaucht wird.

12. Verfahren nach Anspruch 9, worin die Hybridisierung der PNA-Sonde bei einer Temperatur zwischen 53°C und 59°C, bevorzugt 57°C, durchgeführt wird.

13. Verfahren nach einem der Ansprüche 9 bis 12, worin nach der Hybridisierung der PNA-Sonde ein Waschschritt mit einer vorgewärmten Lösung umfassend 5 mM Tris Base; 15 mM NaCl, und; 1 % (Vol./Vol.) Triton X-100, pH 10, durchgeführt wird.

14. Verfahren nach Anspruch 9, worin die Hybridisierung durch Fluoreszenz auftritt.

15. PNA-Sonden wie in einem der Ansprüche 1 bis 4 beschrieben, zur Anwendung zur Detektion von *Salmonella* spp. in biologischen Proben.

16. Das Kit wie in einem der Ansprüche 5 bis 8 beschrieben, zur Anwendung zur Detektion von *Salmonella* spp. in biologischen Proben.

## Revendications

1. Sonde de PNA pour la détection et/ou pour la quantification de *Salmonella,* **caractérisée en ce qu'**elle comprend la séquence SEQ ID No. 1 - 5'-AGG AGC TTC GCT TGC-3'.

2. Sonde de PNA selon la revendication 1, pour la détection de la séquence cible dans l'ARNr, l'ADNr ou les séquences complémentaires de l'ARNr de *Salmonella.*

3. Sonde de PNA selon l'une quelconque des revendications 1 à 2, **caractérisée en ce qu'**elle est liée à au moins un type de fraction détectable, choisie parmi l'un des groupes suivants: un conjugué, un système de détection ramifié, un chromophore, un fluorophore, un radioisotope, une enzyme, un haptène ou un composé luminescent.

4. Sonde de PNA selon la revendication 3, **caractérisé en ce que** ledit groupe fluorophore est au moins l'un des suivants: fluorophores de la série Alexa, de la série Alexa Fluor, cyanines, 5-(et-6)carboxy-2',7'-dichlorofluorescéine, le 5-ROX (5-carboxy-X-rhodamine, sel de triéthylammonium).

5. Kit pour la détection de *Salmonella,* **caractérisé en ce qu'**il comprend au moins une des sondes de PNA décrites dans l'une quelconque des revendications 1 à 4.

6. Kit selon la revendication 5, **caractérisé en ce qu'**il comprend en outre au moins l'une des solutions suivantes: une solution de fixation, une solution d'hybridation et une solution de lavage.

7. Kit selon la revendication 6, dans lequel la solution de fixation comprend de paraformaldéhyde et de l'éthanol, préférablement 2% - 8% (poids/volume) de paraformaldéhyde et de 25% - 90% (volume/volume) d'éthanol.

8. Kit selon la revendication 6, dans lequel la solution d'hybridation comprend du formamide.

9. Procédé pour la détection de *Salmonella,* **caractérisé en ce qu'**il utilise les sondes de PNA décrites dans l'une quelconque des revendications 1 à 4 et qu'il comprend les étapes suivantes :
- contact de la sonde de PNA avec les échantillons biologiques;
- hybridation de la sonde de PNA avec la séquence cible des micro-organismes présents dans les échantillons mentionnés;
- détection de la hybridation comme une indication de la détection et de la quantification mentionnées des échantillons mentionnés ;
dans lequel la hybridation de la sonde de PNA est réalisée par la couverture du échantillon biologique avec une solution de hybridation comprenant: 10% (poids/volume) de sulfate de dextran; 10 mM de NaCl; 30% (volume/volume) de formamide; 0,1% (poids/volume) de pyrophosphate de sodium; 0,2% (poids/volume) de la polyvinylpyrrolidone; 0,2% (poids/volume) de Ficoll; 5 mM d'EDTA disodique; 0,1% (volume/volume) de Triton X-100; 50 mM de Tris-HCl, pH 7,5, et; 200 nM de la sonde de PNA selon l'une quelconque des revendications 1 à 4.

10. Procédé selon la revendication 9, dans lequel l'échantillon biologique est dérivé du sang, de l'air, des aliments, de l'eau ou des biopsies est ne contient pas d'autres acides nucléiques comprenant la séquence cible pour les sondes de PNA décrites dans l'une quelconque des revendications 1 - 4.

11. Procédé selon l'une quelconque des revendications 9 à 10, dans lequel l'échantillon biologique est immergé dans une solution de fixation comprenant de paraformaldéhyde et de l'éthanol, préférablement 2% - 8% (poids/volume) de paraformaldéhyde et de 25% - 90% (volume/volume) d'éthanol.

12. Procédé selon la revendication 9, dans lequel l'hybridation de la sonde de PNA est réalisée à une température comprise entre 53°C et 59°C, préférablement 57°C.

13. Procédé selon l'une quelconque des revendications 9 à 12, dans lequel après l'hybridation de la sonde de PNA est réalisée une étape de lavage avec une solution préchauffée comprenant 5 mM de Tris Base; 15 mM de NaCl, et; 1 % (volume/volume) de Triton X-100, pH 10.

14. Procédé selon la revendication 9, dans lequel l'hybridation est faite par fluorescence.

15. Sondes de PNA telles que décrites dans l'une quelconque des revendications 1 à 4, pour l'utilisation dans la détection de *Salmonella* spp. dans des échantillons biologiques.

16. Le kit tel que décrit dans l'une quelconque des revendications 5 à 8, pour l'utilisation dans la détection de *Salmonella* spp. dans des échantillons biologiques.
